# EUROPEAN PATENT APPLICATION

(11) **EP 4 080 519 A1**
(43) Date of publication of application: **26.10.2022**
(21) Application number: 20903354.7
(22) Date of filing: 08.12.2020
(51) Int. Cl.: G16H 50/50, A61B 5/00, A61B 5/145

(54) **SIMULATION SYSTEM AND PROGRAM**

(30) Priority: 19.12.2019 JP 2019229195
(71) Applicant: Xenlon Corporation, Shinagawa-ku Tokyo 141-0022 (JP); Medical Data Vision Co., Ltd., Chiyoda-ku Tokyo 101-0053 (JP)
(72) Inventor: KOBAYASHI, Kimiyuki, Tokyo 141-0022 (JP)
(74) Representative: TBK
(86) International application number: PCT/JP2020/045649
(87) International publication number: WO 2021/124985

(57) **Abstract**

Provided is a simulation system that gives new awareness to doctors regarding medical diagnosis, and a program product for attaining said system. A simulation system 100 performs a simulation related to a specific disease or symptom for which a major index for diagnosis is assigned, includes: a database 20 created on the basis of inspection results collected from a large number of subjects; a smartphone 30 or a personal computer 40 through which a freely selectable value is entered for each of a plurality of inspection items contained in the inspection results; and a server 10 that collates the value of each inspection item having been entered, with the database 20, and derives a score regarding the specific disease or symptom, wherein the inspection item regarding the major index is preliminarily excluded from the inspection items allowed for entry.

## Description

### TECHNICAL FIELD

This invention relates to a simulation system and a program product.

### BACKGROUND ART

Various techniques have been developed aiming at assisting diagnoses by doctors.

For example, a computer device disclosed in Patent Literature 1 is a device that assists diagnoses of diseases such as diabetes, and displays patients' clinical conditions and physicians' findings in past diagnoses. Now, the patients' clinical conditions displayed on the device contain reproduced values of oral glucose tolerance test (OGTT) reproduced by simulation analysis.

### CITATION LIST

### PATENT LITERATURE

[Patent Literature 1] JP2008-293171A

### SUMMARY OF THE INVENTION

### TECHNICAL PROBLEM

Now, the computer system involved in medical simulation analysis (simply referred to as simulation system, hereinafter), exemplified in Patent Literature 1, is usually on the premise of conducting the simulation analysis on the basis of a logic backed by medical science.

Such premise can only yield analytical results caught in medical common sense, and would occasionally interfere optimum simulation analysis.

This invention was arrived at in consideration of the aforementioned problem, aimed at providing a simulation system capable of giving a new awareness to doctors regarding medical diagnoses, and a program product that attains the system.

### SOLUTION TO PROBLEM

According to a first aspect of this invention, there is provided a simulation system that performs a simulation related to a specific disease or symptom for which a major index for diagnosis is assigned, the simulation system including: a database created on the basis of inspection results collected from a large number of subjects; an entry section through which a freely selectable value is entered for each of a plurality of inspection items contained in the inspection results; and a calculation unit that collates the value of each inspection item having been entered through the entry section, with the database, and derives a score regarding the specific disease or symptom, the inspection item regarding the major index being preliminarily excluded from the inspection items allowed for entry through the entry section.

According to a second aspect of this invention, there is provided a program product that causes a computer to perform a simulation related to a specific disease or symptom for which a major index for diagnosis is assigned, the program product causing the computer to execute: an entry process entering a freely selectable value for each of a plurality of inspection items contained in inspection results regarding the specific disease or symptom; and an output process outputting a score regarding the specific disease or symptom, derived by collating the value of each inspection item entered by the entry process, with a database created on the basis of the inspection results collected from a large number of subjects, the inspection item regarding the major index being preliminarily excluded from the inspection items allowed for entry by the entry process.

According to the above-mentioned invention, an inspection item regarding the major index which is used to diagnose a disease or symptom to be analyzed is preliminarily excluded from the individual inspection items allowed for numerical entry for use in simulation analysis, and result of the entry is collated with a database created on the basis of actual inspection results collected from a large number of subjects, to estimate a score regarding the disease or symptom.

This would occasionally provide an analytical result beyond medical common sense, and if so, can give a new awareness to doctors.

### ADVANTAGEOUS EFFECTS OF INVENTION

This invention can provide a simulation system capable of giving a new awareness to doctors regarding medical diagnosis, and a program product that attains the system.

### BRIEF DESCRIPTION OF DRAWINGS

FIG. 1 is a construction drawing illustrating a simulation system.
FIG. 2 is a sequence diagram regarding processes in the simulation system.
FIG. 3 is a drawing illustrating a part of a display screen regarding the simulation analysis.
FIG. 4 is a graph illustrating score that varies in response to change in entered value regarding white blood cell count.
FIG. 5 is a graph illustrating score that varies in response to change in entered value regarding red blood cell count.
FIG. 6 is a graph illustrating score that varies in response to change in entered value regarding hemoglobin.
FIG. 7 is a graph illustrating score that varies in response to change in entered value regarding hematocrit.

### DESCRIPTION OF EMBODIMENTS

Embodiments of this invention will be explained below referring to the attached drawings. Note that all similar constituents in all drawings will have the same reference signs to properly skip the explanation as appropriate.

### <Structure of Simulation System>

First, a structure of a simulation system 100 of this embodiment will be explained.

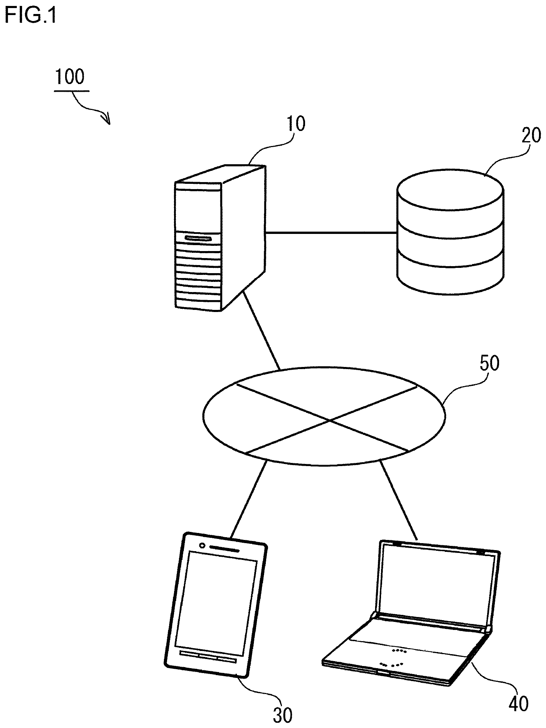

FIG. 1 is a construction drawing of the simulation system 100.

The simulation system 100 of this embodiment performs simulation analysis and quantification of diabetes-related risk.

Now, the diabetes-related risk can for example mean onset possibility for non-progressors of diabetes, and the severity for progressors of diabetes.

Quantified diabetes-related risk will be referred to as "score" for convenience.

The simulation system 100 has a server 10, a database 20, a smartphone 30, and a personal computer 40.

The server 10 is configured as to be able to communicate with the smartphone 30 and the personal computer 40 through a network 50. The network 50 is a computer network typically constructed by the Internet, mobile phone communication network, or LAN (local area network).

The server 10 is configured as to be able to access the database 20, and can derive the score referring to the database 20.

The database 20 is created by subjecting a large volume of inspection results collected from a large number of subjects, to data cleansing on the basis of a unique logic, followed by so-called big data analysis (various database technologies, deep learning, etc.).

The database 20 may only be the one created on the basis of actual inspection result, and may be embodied by any combination of known techniques without special limitation.

The smartphone 30 and the personal computer 40 are examples of computer devices positioned as client terminals for the server 10.

The smartphone 30 and the personal computer 40 are made accessible to the server 10 by installing application program products describe later, and can request the score derived by the server 10.

The application program products to be installed on the smartphone 30 and the personal computer 40, in this embodiment, are the products responsible for execution of simulation regarding diabetes.

Each application program product is stored in each storage (not illustrated) in the smartphone 30 or the personal computer 40, and can cause the smartphone 30 or the personal computer 40 to execute an entry process and a output process explained next, after read out by each CPU (not illustrated) in the smartphone 30 or the personal computer 40.

Now the entry process refers to a process conducted by the user who enters a freely selectable value to each of a plurality of inspection items contained in inspection results regarding diabetes.

Meanwhile the output process refers to a process of outputting a diabetes-related score in a format recognizable by the user, the diabetes-related score being derived by collating the value of each inspection item entered by the entry process with the database 20.

Details of the inspection item to be entered by the entry process, and the details of the score obtained as a result of entry will be described later.

### <Process Procedures in Simulation System>

Next, process procedures in the simulation system 100 of this embodiment will be explained.

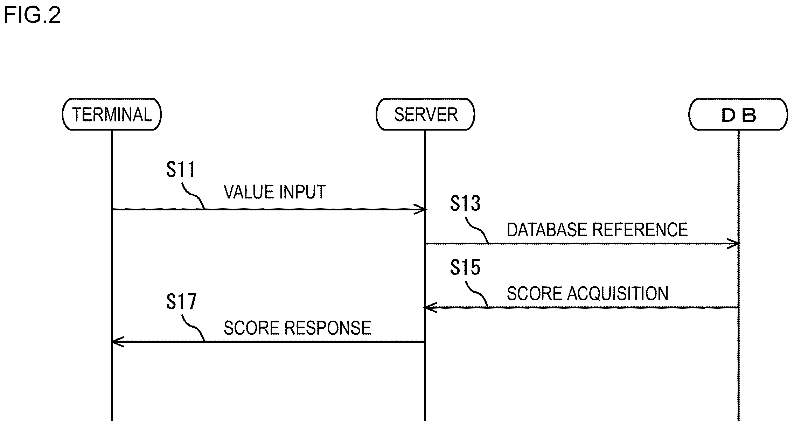

FIG. 2 is a sequence diagram regarding processes in the simulation system 100.

The explanation below will be given on the premise that a "terminal" in FIG. 2 corresponds to the smartphone 30 in FIG. 1, a "server" in FIG. 2 corresponds to the server 10 in FIG. 1, and a "DB" in FIG. 2 corresponds to the database 20 in FIG. 1. Note that the "terminal" in FIG. 2 will keep the process procedures unchanged, even if replaced with the personal computer 40 in FIG. 1.

First, the user enters a value of an inspection result, for each of inspection items in inspection for diagnosing diabetes (blood test, for example), with use of an entry section in the form of slide bar displayed on a display screen of the smartphone 30 (step S11).

The value entered in step S11 is sent from the smartphone 30 to the server 10, and the server 10 collates the value with the database 20 (step S13).

The server 10 can acquire a score regarding diabetes, as a result of collation in step S13 (step S15).

The server 10 responds to the smartphone 30 with the score acquired in step S15 (step S17).

Since the above-described process procedures is conducted every time the value to be entered through the display screen (entry section in the form of slide bar) of the smartphone 30 is changed, so that the user can check changes in the score while varying the value entered through the display screen.

### <Display Screen Regarding Simulation Analysis>

Next, the display screen regarding simulation analysis conducted by the simulation system 100 (the display screens of the smartphone 30 and the personal computer 40) will be explained.

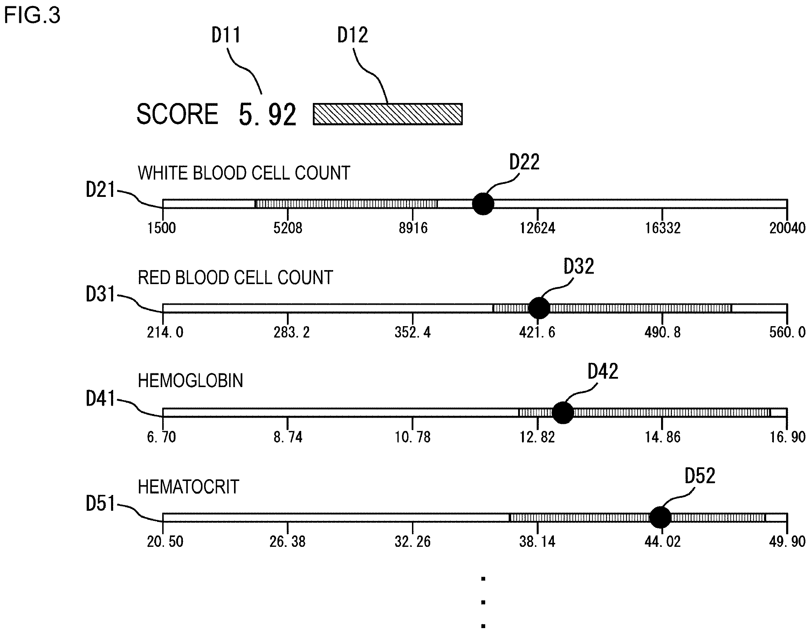

FIG. 3 is a drawing illustrating a part of a display screen regarding the simulation analysis. Note that a mode of display illustrated in FIG. 3 is a specific example, so that embodiment of this invention is not limited thereto.

Display D11 is a numerical presentation of the score regarding diabetes. Although this embodiment will be explained on the premise that the larger the value, the higher the diabetes-related risk, and that the smaller the value, the lower the diabetes-related risk, the relationship may be inverted. That is, this invention may be embodied in a mode on the premise that the smaller the value, the higher the diabetes-related risk, and that the larger the value, the lower the diabetes-related risk.

Display D12 is a band whose length can vary in proportion to the value of display D11. Display D12 also expresses the magnitude of risk indicated by the value of display D11 with its color. More specifically, display D12 turns red for high risk, turns yellow for medium risk, and turns green for low risk.

Display D21 represents an entry section in the form of slide bar that accepts numerical entry of an inspection result regarding white blood cell count, on which the value to be entered can vary with lateral movement of a slider D22.

A numerical range allowed for entry on display D21 is specified from 1,500/µL to 20,040/µL. Display D21 contains a standard range of the white blood cell count (shaded part in display D21 in FIG. 3), whose display mode is discriminable from the residual range. The standard range of the white blood cell count in display D21 is specified from 3,600/µL to 9,000/µL.

Display D31 represents an entry section in the form of slide bar that accepts numerical entry of an inspection result regarding red blood cell count, on which the value to be entered can vary with lateral movement of a slider D32.

A numerical range allowed for entry on display D31 is specified from 2,140,000/µL to 5,600,000/µL. Display D31 contains a standard range of the red blood cell count (shaded part in display D31 in FIG. 3), whose display mode is discriminable from the residual range. The standard range of the red blood cell count in display D31 is specified from 3,870,000/µL to 5,250,000/µL.

Display D41 represents an entry section in the form of slide bar that accepts numerical entry of an inspection result regarding hemoglobin, on which the value to be entered can vary with lateral movement of a slider D42.

A numerical range allowed for entry on display D41 is specified from 6.7 g/dL to 16.9 g/dL. Display D41 contains a standard range of hemoglobin (shaded part in display D41 in FIG. 3), whose display mode is discriminable from the residual range. The standard range of hemoglobin in display D41 is specified from 12.6 g/dL to 16.5 g/dL.

Display D51 represents an entry section in the form of slide bar that accepts numerical entry of an inspection result regarding hematocrit, on which the value to be entered can vary with lateral movement of a slider D52.

A numerical range allowed for entry on display D51 is specified from 20.5% to 49.9%. Display D51 contains a standard range of hematocrit (shaded part in display D51 in FIG. 3), whose display mode is discriminable from the residual range. The standard range of hematocrit in display D51 is specified from 37.4% to 48.6%.

The numerical ranges allowed for entry, and the standard ranges of the individual inspection items on display D21 to display D51 are merely illustrative ones, and may be modified as appropriate. In particular, the individual inspection items will have appropriate ranges which differ depending on gender of the user, and are therefore preferably modifiable depending on the gender of the user.

The display screen also contains entry sections allowed for entry of inspection results regarding the inspection items below, although not illustrated in FIG. 3.

The inspection items include platelet count, total protein, albumin, creatine kinase, GOT, GPT, LDH, alkaline phosphatase, γ-GTP, creatinine, uric acid, urea nitrogen, glucose, triglyceride, sodium, potassium, chlorine, total bilirubin, CRP, and estimated GFRcreat. These inspection items and four inspection items illustrated in FIG. 3 (white blood cell count, red blood cell count, hemoglobin, and hematocrit) are picked up as inspection targets which are often involved in usual blood test.

Now the simulation system 100 of this embodiment is featured by that HbAlc (hemoglobin a-one-c), which is the most important index for diagnosing diabetes, has been excluded from the inspection items allowed for entry of the inspection results on the display screen.

Such feature enables the simulation system 100 to derive a conclusion that is not obtainable from usual diagnoses, and can provide a new awareness to doctors.

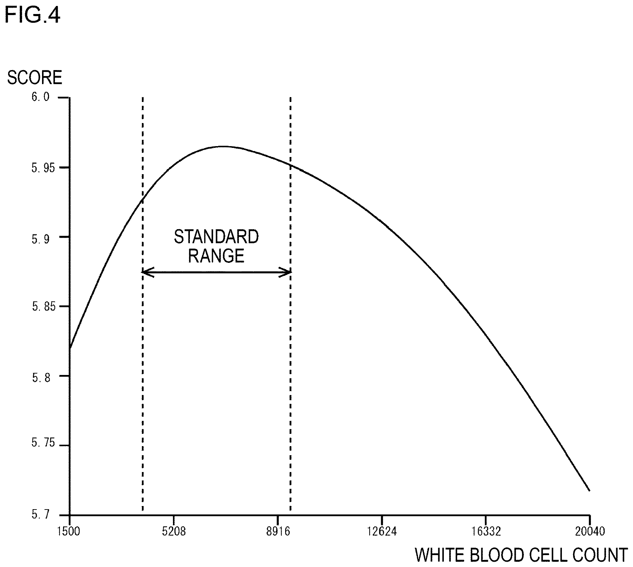

Paragraphs below will describe, referring to FIG. 4 to FIG. 7, how the score varies, in response to change of value to be entered within an allowable range for the entry, for one inspection item out of the four inspection items (white blood cell count, red blood cell count, hemoglobin, and hematocrit) illustrated in the FIG. 3.

Note that the variations of the scores illustrated in FIG. 4 to FIG. 7 can result from interaction among the inspection results (numerical values) entered for the individual inspection items, whose tendencies are demonstrated not in common for all users. In other words, tendencies of variation in the score can vary for every inspection result to be entered.

FIG. 4 is a graph illustrating score that varies in response to change in entered value regarding the white blood cell count.

As illustrated in FIG. 4, when varying the entered values of the white blood cell count within the range from 1,500/µL to 20,040/µL, the maximum score (worst score) falls in the standard range. Meanwhile, upon entry within the aforementioned range, the best score falls out of the standard range.

Although the standard range of white blood cell count is considered to be normal in usual blood test, the results teach that values out of the standard range would be better, from the viewpoint of risk determination regarding a specific disease such as diabetes as in this embodiment.

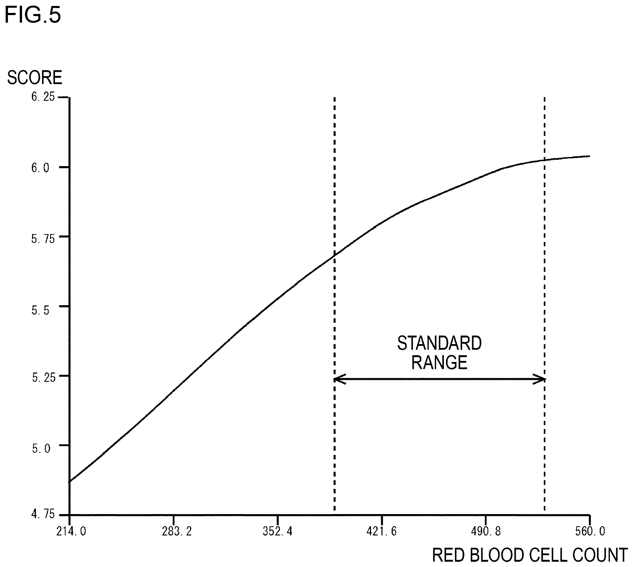

FIG. 5 is a graph illustrating score that varies in response to change in entered value regarding the red blood cell count.

As illustrated in FIG. 5, when varying the entered values of the red blood cell count within the range from 2,140,000/µL to 5,600,000/µL, a change of the score change is a positive correlation with the red blood cell count. Hence, the maximum score (worst score) corresponds to a value (6.06) obtained upon entry of 5,600,000/µL. On the other hand, the minimum score (best score) corresponds to a value (4.87) obtained upon entry of 2,140,000/µL. Hence, upon entry within the aforementioned range, the best score falls out of the standard range.

Similarly to the standard range for the white blood cell count, also the standard range for the red blood cell count is considered to be normal in usual blood test. The results, however, teach that the lesser the red blood cell count the better, from the viewpoint of risk determination regarding a specific disease such as diabetes as in this embodiment.

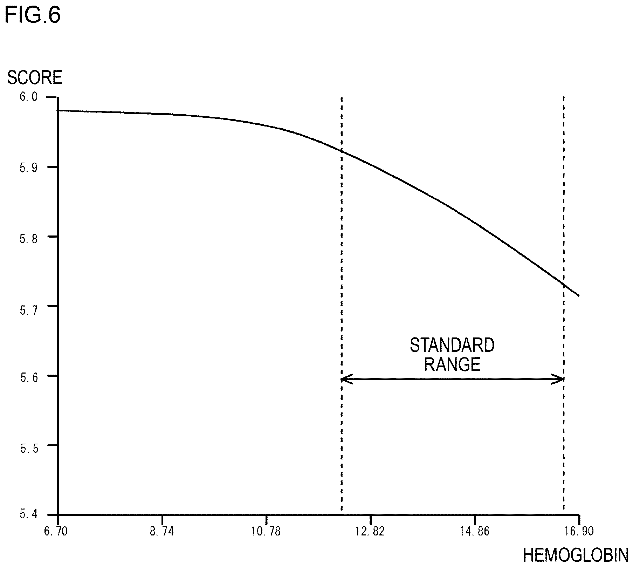

FIG. 6 is a graph illustrating score that varies in response to change in entered value regarding the hemoglobin.

As illustrated in FIG. 6, when varying the entered values of the hemoglobin within the range from 6.7 g/dL to 16.9 g/dL, a change of the score is a negative correlation with the hemoglobin. Hence, the maximum score (worst score) corresponds to a value (5.72) obtained upon entry of 16.9g/dL. On the other hand, the minimum score (best score) corresponds to a value (5.98) obtained upon entry of 6.7 g/dL. Hence, upon entry within the aforementioned range, the best score falls out of the standard range.

Similarly to the standard ranges for the red blood cell and for the white blood cell count, also the standard range for the hemoglobin is considered to be normal in usual blood test. The results, however, teach that the more the hemoglobin the better, from the viewpoint of risk determination regarding a specific disease such as diabetes as in this embodiment.

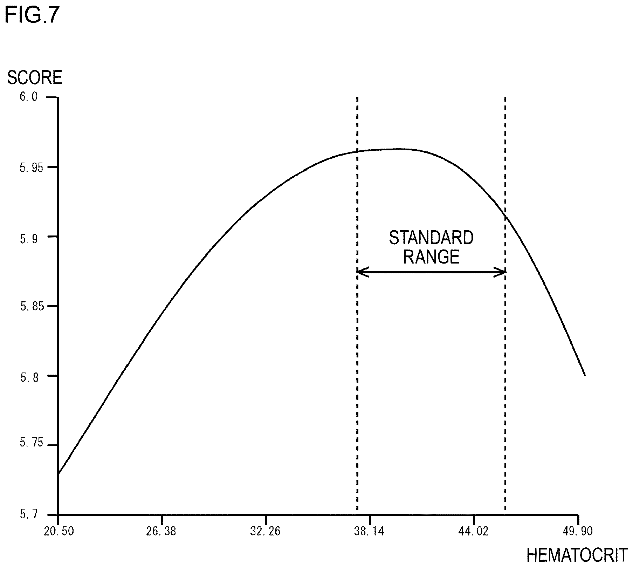

FIG. 7 is a graph illustrating score that varies in response to change in entered value regarding the hematocrit.

As illustrated in FIG. 7, when varying the entered values of the hematocrit within the range from 20.5% to 49.9%, the maximum score (worst score) falls in the standard range. Meanwhile, upon entry within the aforementioned range, the best score falls out of the standard range.

Similarly to the standard ranges for the red blood cell, white blood cell count, and hemoglobin, also the standard range for the hematocrit is considered to be normal in usual blood test. The results, however, teach that values out of the standard range would be better, from the viewpoint of risk determination regarding a specific disease such as diabetes as in this embodiment.

As is clear from the tendencies of variation in the score illustrated in FIG. 4 to FIG. 7, the simulation system 100 occasionally affirms the values which are not recommendable (judged to be abnormal) in usual blood test, and can give a new awareness to doctors.

Next paragraphs will explain findings obtainable in this invention, from a viewpoint different from the aforementioned tendencies in score changes.

As for GOT and GPT, both being inspection items used for diagnosing liver function, the former demonstrates a decreasing tendency of the score as the value increases, meanwhile the latter demonstrates an increasing tendency of the score as the value increases. Hence, from the viewpoint of risk determination regarding a specific disease such as diabetes, the higher the GOT the better, and the lower the GPT the better. This teaches that the GOT and GPT need different points of view when determining risk of diabetes.

Creatine, which is an inspection item used for diagnosing kidney function, more greatly affects the score as compared with other inspection items used for diagnosing kidney function (total protein, albumin, uric acid, urea nitrogen, estimated GFRcreat), demonstrating a decreasing tendency of the score as the creatin value increases. Hence, the creatine can be an important index, also from the viewpoint of risk determination regarding a specific disease such as diabetes.

### <Conclusion>

The aforementioned simulation system 100 will be summarized below.

The simulation system 100 performs a simulation related to diabetes, wherein HbAlc (hemoglobin a-one-c) is used as a major index in an usual diagnosis of diabetes.

The database 20 is created on the basis of inspection results collected from a large number of subjects, and corresponds to the "database" in this invention.

The display screen displayed on the smartphone 30 or the personal computer 40 (display screen in FIG. 3) has an entry section in the form of slide bar, allowed for entry of a freely selectable value for each of a plurality of inspection items contained in the inspection results, and corresponds to the "entry section" in this invention.

The server 10 collates the value of each inspection item having been entered, with the database, and derives a score regarding the specific disease or symptom, and therefore corresponds to the "calculation unit" in this invention.

The application program product to be installed on the smartphone 30 or the personal computer 40 is a program product that causes a computer to perform a simulation regarding diabetes which employs, in the usual diagnosis, HbAlc (hemoglobin a-one-c) as a major index.

The program product causes the smartphone 30 or the personal computer 40 to execute the aforementioned entry process and output process.

The simulation system 100 and the application program product commonly have a feature that HbAlc (hemoglobin a-one-c), which is an inspection item regarding the major index, has been excluded from the inspection items allowed for entry on the entry section (or in the entry process).

By virtue of such feature, the simulation system 100 and the application program product can give a new awareness to doctors, without being caught up in the medical common sense.

### <Modified Example>

The aforementioned embodiment is merely an illustrative one of possible embodiments of this invention, and may be modified in various ways so long as the purpose of this invention will be achieved.

Although the aforementioned embodiment exemplified the case where the "database", "entry section", and "calculation unit" in this invention are embodied as separate devices, two or three of the structures may alternatively be embodied as an integrated device.

Although FIG. 1 illustrated each of the structures that correspond to the" database" and "calculation unit" in this invention as a single device, each of them may alternatively be embodied by a plurality of devices.

Although the aforementioned embodiment exemplified the case where the "entry process" and "output process" in this invention are embodied on the same display screen, the processes may alternatively be embodied on different display screens.

The "output process" in this invention is not always necessarily given in the form of display output, but may alternatively be in the form of print output or audio output.

Although the aforementioned embodiment explained the case where the disease to be subjected to simulation analysis was diabetes, any other disease or symptom may alternatively be subjected to the simulation analysis on the basis of the same technical spirit.

For example, in a case where the disease or symptom to be subjected to simulation analysis is at least either polycythemia or erythrocythemia, the inspection item regarding the major index, to be preliminarily excluded from the inspection items allowed for entry, is preferably at least one of red blood cell count, hemoglobin or hematocrit.

In a case where the disease or symptom to be subjected to simulation analysis is eosinophilia, the inspection item regarding the major index, to be preliminarily excluded from the inspection items allowed for entry, is preferably eosinophil count.

In a case where the disease or symptom to be subjected to simulation analysis is high triglycerides, the inspection item regarding the major index, to be preliminarily excluded from the inspection items allowed for entry, is preferably triglyceride.

In a case where the disease or symptom to be subjected to simulation analysis is high LDL cholesterol, the inspection item regarding the major index, to be preliminarily excluded from the inspection items allowed for entry, is preferably LDL cholesterol.

In a case where the disease or symptom to be subjected to simulation analysis is myocardial infarction, the inspection item regarding the major index, to be preliminarily excluded from the inspection items allowed for entry, is preferably cardiac troponin I (cTnI).

In a case where the disease or symptom to be subjected to simulation analysis is hepatitis B, the inspection item regarding the major index, to be preliminarily excluded from the inspection items allowed for entry, is preferably HBV-DNA quantification.

In a case where the disease or symptom to be subjected to simulation analysis is hepatitis C, the inspection item regarding the major index, to be preliminarily excluded from the inspection items allowed for entry, is preferably HCV-RNA quantification.

In a case where the disease or symptom to be subjected to simulation analysis is renal dysfunction, the inspection item regarding the major index, to be preliminarily excluded from the inspection items allowed for entry, is preferably estimated GFRcreat.

The aforementioned embodiment exemplified the case where the diabetes-related score was given on the display screen, in the form of numerical display D11 and the bar-like display D12 that varies the length in proportion to the numerical value, wherein either one of them is omissible, or any other display may be presented together.

For example, the display output may contain, in addition to the aforementioned display, advice or therapeutic policy (comments on diet, exercise, or drug therapy, etc.) for improving the score.

Alternatively, the display output may contain any inspection item to be focused for improving the score, and a target level of the inspection item. In such modified example, the inspection item to be focused (inspection item to which a target level is determined) is preferably the one capable of more largely improving the score, as compared with any other inspection items when varied to the same degree.

This embodiment encompasses the technical spirits below.
(1) A simulation system that performs a simulation related to a specific disease or symptom for which a major index for diagnosis is assigned, the simulation system including: a database created on the basis of inspection results collected from a large number of subjects; an entry section through which a freely selectable value is entered for each of a plurality of inspection items contained in the inspection results; and a calculation unit that collates the value of each inspection item having been entered through the entry section, with the database, and derives a score regarding the specific disease or symptom, the inspection item regarding the major index being preliminarily excluded from the inspection items allowed for entry through the entry section.
(2) The simulation system according to (1), wherein the specific disease or symptom is diabetes, and the inspection item regarding the major index, being preliminarily excluded from the inspection items allowed for entry through the entry section, is HbAlc (hemoglobin a-one-c).
(3) The simulation system according to (1), wherein the specific disease or symptom is at least either polycythemia or erythrocythemia, and the inspection item regarding the major index, being preliminarily excluded from the inspection items allowed for entry through the entry section, is at least one of red blood cell count, hemoglobin or hematocrit.
(4) The simulation system according to (1), wherein the specific disease or symptom is eosinophilia, and the inspection item regarding the major index, being preliminarily excluded from the inspection items allowed for entry through the entry section, is eosinophil count.
(5) The simulation system according to (1), wherein the specific disease or symptom is high triglycerides, and the inspection item regarding the major index, being preliminarily excluded from the inspection items allowed for entry through the entry section, is triglyceride.
(6) The simulation system according to (1), wherein the specific disease or symptom is high LDL cholesterol, and the inspection item regarding the major index, being preliminarily excluded from the inspection items allowed for entry through the entry section, is LDL cholesterol.
(7) The simulation system according to (1), wherein the specific disease or symptom is myocardial infarction, and the inspection item regarding the major index, being preliminarily excluded from the inspection items allowed for entry through the entry section, is cardiac troponin I (cTnI).
(8) The simulation system according to (1), wherein the specific disease or symptom is hepatitis B, and the inspection item regarding the major index, being preliminarily excluded from the inspection items allowed for entry through the entry section, is HBV-DNA quantification.
(9) The simulation system according to (1), wherein the specific disease or symptom is hepatitis C, and the inspection item regarding the major index, being preliminarily excluded from the inspection items allowed for entry through the entry section, is HCV-RNA quantification.
(10) The simulation system according to (1), wherein the specific disease or symptom is renal dysfunction, and the inspection item regarding the major index, being preliminarily excluded from the inspection items allowed for entry through the entry section, is estimated GFRcreat.
(11) The simulation system according to any one of (1) to (10), wherein a standard range is specified for each inspection item which is at least a part of the inspection items allowed for entry through the entry section, the score derived by the calculation unit varies in response to change in each inspection item entered through the entry section, and when a value to be entered regarding one inspection item varies, a value that gives the best score may fall out of the standard range.
(12) A program product that causes a computer to perform simulation related to a specific disease or symptom for which a major index for diagnosis is assigned, the program product causing the computer to execute: an entry process entering a freely selectable value for each of a plurality of inspection items contained in inspection results regarding the specific disease or symptom; and an output process outputting a score regarding the specific disease or symptom, derived by collating the value of each inspection item entered by the entry process, with a database created on the basis of the inspection results collected from a large number of subjects, the inspection item regarding the major index being preliminarily excluded from the inspection items allowed for entry by the entry process.

This application claims priority to Japanese Patent Application No. 2019-229195 filed on December 19, 2019, the entire contents of which are incorporated by reference herein.

### REFERENCE SIGSN LIST

- 100: simulation system
- 10: server
- 20: database
- 30: smartphone
- 40: personal computer
- 50: network

## Claims

1. A simulation system that performs a simulation related to a specific disease or symptom for which a major index for diagnosis is assigned, the simulation system comprising:
a database created on the basis of inspection results collected from a large number of subjects;
an entry section through which a freely selectable value is entered for each of a plurality of inspection items contained in the inspection results; and
a calculation unit that collates the value of each inspection item having been entered through the entry section, with the database, and derives a score regarding the specific disease or symptom,
the inspection item regarding the major index being preliminarily excluded from the inspection items allowed for entry through the entry section.

2. The simulation system according to claim 1,
wherein the specific disease or symptom is diabetes, and
the inspection item regarding the major index, being preliminarily excluded from the inspection items allowed for entry through the entry section, is HbAlc (hemoglobin a-one-c).

3. The simulation system according to claim 1,
wherein the specific disease or symptom is at least either polycythemia or erythrocythemia, and
the inspection item regarding the major index, being preliminarily excluded from the inspection items allowed for entry through the entry section, is at least one of red blood cell count, hemoglobin or hematocrit.

4. The simulation system according to claim 1,
wherein the specific disease or symptom is eosinophilia, and
the inspection item regarding the major index, being preliminarily excluded from the inspection items allowed for entry through the entry section, is eosinophil count.

5. The simulation system according to claim 1,
wherein the specific disease or symptom is high triglycerides, and
the inspection item regarding the major index, being preliminarily excluded from the inspection items allowed for entry through the entry section, is triglyceride.

6. The simulation system according to claim 1,
wherein the specific disease or symptom is high LDL cholesterol, and
the inspection item regarding the major index, being preliminarily excluded from the inspection items allowed for entry through the entry section, is LDL cholesterol.

7. The simulation system according to claim 1,
wherein the specific disease or symptom is myocardial infarction, and
the inspection item regarding the major index, being preliminarily excluded from the inspection items allowed for entry through the entry section, is cardiac troponin I (cTnI).

8. The simulation system according to claim 1,
wherein the specific disease or symptom is hepatitis B, and
the inspection item regarding the major index, being preliminarily excluded from the inspection items allowed for entry through the entry section, is HBV-DNA quantification.

9. The simulation system according to claim 1,
wherein the specific disease or symptom is hepatitis C, and
the inspection item regarding the major index, being preliminarily excluded from the inspection items allowed for entry through the entry section, is HCV-RNA quantification.

10. The simulation system according to claim 1,
wherein the specific disease or symptom is renal dysfunction, and
the inspection item regarding the major index, being preliminarily excluded from the inspection items allowed for entry through the entry section, is estimated GFRcreat.

11. The simulation system according to any one of claims 1 to 10,
wherein a standard range is assigned for each inspection item which is at least a part of the inspection items allowed for entry through the entry section,
the score derived by the calculation unit varies in response to change in value of each inspection item entered through the entry section, and
when a value to be entered regarding one inspection item varies, a value that gives the best score may fall out of the standard range.

12. A program product that causes a computer to perform a simulation related to a specific disease or symptom for which a major index for diagnosis is assigned, the program product causing the computer to execute:
an entry process entering a freely selectable value for each of a plurality of inspection items contained in inspection results regarding the specific disease or symptom; and
an output process outputting a score regarding the specific disease or symptom, derived by collating the value of each inspection item entered by the entry process, with a database created on the basis of the inspection results collected from a large number of subjects,
the inspection item regarding the major index being preliminarily excluded from the inspection items allowed for entry by the entry process.
